# EUROPEAN PATENT APPLICATION

(11) **EP 4 471 015 A1**
(43) Date of publication of application: **04.12.2024**
(21) Application number: 23305864.3
(22) Date of filing: 01.06.2023
(51) Int. Cl.: C07D 277/64, C07D 277/68, C07D 277/74, C07D 277/82

(54) **PROCESS FOR THE SELECTIVE CLEAVAGE OF C-SCF3 BONDS AND ANALOGUES**

(71) Applicant: Centre National de la Recherche Scientifique, 75016 Paris (FR); Institut National des Sciences Appliquées de Rouen Normandie, 76801 Saint-Etienne-du-Rouvray, Cedex (FR); Université de Rouen Normandie, 76130 Mont-Saint-Aignan (FR)
(72) Inventor: BESSET, Tatiana, 76230 ISNEAUVILLE (FR); CASTANHEIRO Matias, Thomas, 76770 MALAUNAY (FR); NOBILE, Enzo, 91300 MASSY (FR); ARRIBAT, Mathieu, 27460 ALIZAY (FR)
(74) Representative: Brevalex

(57) **Abstract**

The present invention relates to a process for the preparation of a compound A-R, by reacting a compound A-X, in the presence of a metallic complex comprising nickel, a ligand, a nucleophile carrying one group R, and a solvent,
R being a hydrocarbon group, optionally including at least one heteroatom and/or at least one atom other than C or H,
A being selected from the group consisting of: (C₆-C₁₀)aryl groups, heteroaryl groups, and a vinyl compound, and
X being selected from the group consisting of: YCF₃, Y(O)ₙCF₃, YCF₂SO₂Ph, Y(O)ₙCF₂SO₂Ph, Y(O)ₙCF₂COOR, Y(O)ₙCF₂CONR₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR, Y being S or Se, n being 1 or 2, and R being (C₁-C₆)alkyl.

## Description

The present invention concerns a process for the selective cleavage of C-SCF₃ bonds and analogues of such bonds.

In a society concerned about the environment, earth and aquatic systems pollution and circular economy, development of new technologies to meet such objectives is nowadays a real challenge and a hot topic across the whole chemical industry.

The field of organofluorine chemistry is unavoidable and fluorinated compounds are highly represented in many fields such as materials science, pharmaceutical and agrochemical industries. Indeed, the incorporation of a fluorine atom or a fluorinated unit can modify the physico-chemical properties of organic molecules in a significant way explaining the interest of the scientific community regarding this research area. However, the prevalence of these compounds raises the question of their fate and degradation. Recently, there has been a strong awareness from the scientific community and society regarding chlorofluorocarbons (CFCs), hydrofluorocarbons (HFCs) and per- and poly-fluoroalkyl substances (PFAs). It is now important to go even further and to look at the fate of emerging fluorinated groups that have become essential, such as derivatives containing a SCF₃ unit (eg. Toltrazuril and Fipronil).

In this context, the development of tools allowing to valorize these fluorinated derivatives, which, once used, are then considered as waste by converting them into compounds with high added value would have an undeniable ecological impact and allow for cost reduction. It is therefore essential to develop innovative tools to meet this challenge and to remove this synthetic lock.

There is currently no technology allowing to selectively cleave C(sp²)-SRf bonds on (hetero)aromatic derivatives or vinyl positions or C(sp³)-SRf bonds and any progress would have a major impact from a scientific and environmental point of view.

One aim of the present invention is thus to provide a tool for the de-fluorination of fluorinated compounds.

One aim of the present invention is to provide a process for the de-fluorination of fluorinated compounds, for example through the cleavage of C-SCF₃ bonds, or analogues of such bonds.

Thus, the present invention relates to a process for the preparation of a compound having the formula (I):

A-R

by reacting a compound having the formula (II):

   A-X
in the presence of a metallic complex comprising nickel, a ligand, a nucleophile carrying one group R, and a solvent,
R being a hydrocarbon group, optionally including at least one heteroatom such as S, N or O, and/or at least one atom other than C or H, such as Se, Si, B, or P,
wherein:
   - A is selected from the group consisting of:
      . (C₆-C₁₀)aryl groups,
         said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
         Rₐ and R_{b} being independently from each other H or a (C₁-C₆)alkyl group;
         R_{c} being a (C₁-C₆)alkyl group;
      . heteroaryl groups comprising from 5 to 10 atoms and including at least one heteroatom selected from O, N, and S,
         said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
      . a vinyl compound having the following formula (III): 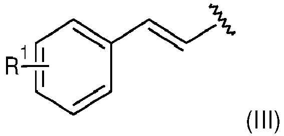 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
   - X is selected from the group consisting of: YCF₃, YOCF₃, YO₂CF₃, YCF₂SO₂Ph, YOCF₂SO₂Ph, YO₂CF₂SO₂Ph, Y(O)ₙCF₂COOR', Y(O)ₙCF₂CONR'₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR', Y being S or Se,
      n being 1 or 2, and R' being (C₁-C₆)alkyl.

According to an embodiment, in formula (II) above, X is selected from the group consisting of: SCF₃, SOCF₃, SO₂CF₃, SCF₂SO₂Ph, SOCF₂SO₂Ph, SO₂CF₂SO₂Ph, S(O)ₙCF₂COOR, S(O)ₙCF₂CONR₂, S(O)ₙCF₂CH₂OH, S(O)ₙCN, S(O)ₙCHF₂, S(O)ₙCF₂H, S(O)ₙCF₂CₙF₂ₙ₊₁, S(O)ₙCF₂COR, and all analogues of this list in the selenium series, R and n being as defined above.

According to an embodiment, in formula (II) above, X is selected from the group consisting of: SCF₃, SOCF₃, SO₂CF₃, SCF₂SO₂Ph, SOCF₂SO₂Ph, SO₂CF₂SO₂Ph, S(O)ₙCF₂COOR, S(O)ₙCF₂CONR₂, S(O)ₙCF₂CH₂OH, S(O)ₙCN, S(O)ₙCHF₂, S(O)ₙCF₂H, S(O)ₙCF₂CₙF₂ₙ₊₁, S(O)ₙCF₂COR, and SeCF₃.

According to an embodiment, in formula (II) above, X is selected from the group consisting of: SCF₃, SOCF₃, SO₂CF₃, SCF₂SO₂Ph, SO₂CF₂SO₂Ph, and SeCF₃.

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl group" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

When an alkyl group is substituted with an aryl group, the term "arylalkyl" or "aralkyl" group is used. The "arylalkyl" or "aralkyl" groups are aryl-alkyl- groups, the aryl and alkyl groups being as defined above. Among the arylalkyl groups, mention may in particular be made of the benzyl or phenethyl groups.

The term "halogen" means: a fluorine, a chlorine, a bromine or an iodine.

The term "alkoxy group" means: an -O-alkyl group where the alkyl group is as previously defined. By way of examples, mention may be made of -O-(C₁-C₄)alkyl groups, and in particular the -O-methyl group, the -O-ethyl group, as -O-Csalkyl group, the -O-propyl group, the -O-isopropyl group, and as -O-C₄alkyl group, the -O-butyl, - O-isobutyl or -O-tert-butyl group.

The term "alkynyl" as employed herein includes unsaturated, nonaromatic, hydrocarbon groups having 2 to 6 carbons, and comprising at least one triple bond. Preferably, the alkynyl group is linear. Preferably, the alkynyl group is a -(CH₂)ₘ-C≡CH group, m being an integer comprised from 1 to 4.

The term "alkenyl" as employed herein includes unsaturated, nonaromatic, hydrocarbon groups having 2 to 6 carbons, and comprising at least one double bond. Preferably, the alkenyl group is linear. Preferably, the alkenyl group is a -(CH₂)ₘ-CH=CH₂ group, m being an integer comprised from 1 to 4.

The abovementioned "alkyl", "aryl", and "heteroaryl" groups can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "haloalkyl group" means: an alkyl group as defined above, in which one or more of the hydrogen atoms is(are) replaced with a halogen atom. By way of example, mention may be made of fluoroalkyls, in particular CF₃ or CHF₂.

The term "carboxyl" means: a COOH group.

The term "oxo" means: "=O".

The term "hydrocarbon group" means a group comprising hydrogen and carbon atoms. As mentioned above, R is a group comprising at least one carbon atom and at least one hydrogen atom. This group may also include at least one heteroatom, such as O, N or S, and/or at least one atom other than C or H, such as Se, Si, B or P.

In one embodiment of the process according to the invention, the nucleophile is selected from the group consisting of: alkyl amines, cycloalkyl amines, aryl amines, alkyl thiols, thiophenol compounds, aryl selenol compounds, phosphine oxides, and Grignard reagents.

Preferably, the nucleophile is a RH group, wherein R is selected from the group consisting of: (C₁-C₆)alkyl, heterocycloalkyl, -S-cyclo(C₃-C₁₀)alkyl, -P(=O)-R¹R², and - X¹-Ar¹, X¹ being S, Se or NH, and Ar¹ being an optionally substituted (C₆-C₁₀)aryl group, and R¹ and R² being, independently from each other, a (C₁-C₆)alkyl group or a (C₆-C₁₀)aryl group.

Preferably, R is selected from the group consisting of: (C₁-C₆)alkyl, heterocycloalkyl, -S-cyclo(C₃-C₁₀)alkyl, -P(=O)-R¹R², and -X¹-Ar¹, X¹ being S, Se or NH, and Ar¹ being an optionally substituted (C₆-C₁₀)aryl group, and R¹ and R² being, independently from each other, a (C₁-C₆)alkyl group or a (C₆-C₁₀)aryl group.

More preferably, the nucleophile is selected from the group consisting of: heterocycloalkyl groups comprising at least one nitrogen and/or sulfur atom such as morpholine, aniline groups and derivatives thereof, such as 4-methylaniline, H-S-(cyclo)alkyl groups, such as CySH, H-S-aryl groups, such as 4-methylthiophenol, H-Se-aryl groups, such as benzene selenol, phosphine oxides such as HP(=O)Ph₂, and Grignard reagents such as Alk-MgBr, in particular iPrMgBr.

In one embodiment, in formula (I) and in formula (II) as defined above, A is selected from the heteroaryl groups comprising from 5 to 10 atoms and including at least one heteroatom selected from O, N, and S, said heteroaryl groups being optionally substituted with at least one substituent selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above.

According to one embodiment, the process of the invention as defined above further comprises the use of a hydride compound, such as LiHMDS. In this embodiment, the compound having the formula (II) is reacted with the nucleophile in the presence of the metallic complex comprising nickel, the ligand, and the solvent, and also with a hydride compound.

In this embodiment, the nucleophile is in particular a Grignard reactant.

According to one embodiment, in the process according to the invention, the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-tert-butylphosphino)ferrocene (DTBPF), 4,5-Bis(diphenylphosphino)-9,9-dimethylxanthene (XantPhos), and triphenylarsine (AsPh₃).

Preferably, the metallic complex is a Ni(0) complex such as bis(1,5-cyclooctadiene)nickel.

According to one embodiment, the solvent used in the process according to the invention is selected from the usual solvents used in the field. Preferably, the solvent for the process according to the invention is toluene.

According to one embodiment of the process according to the invention, the reaction is carried out a temperature from 90°C to 140°C. Preferably, the reaction may be carried out at 90°C, 120°C, and 140°C.

The present invention also relates to the process as defined above, for the preparation of a compound having the formula (I-1): 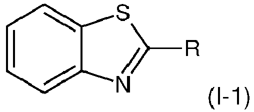
R being as defined above,
from a compound having the formula (II-1):

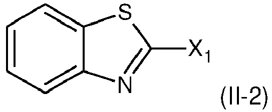

X₁ being selected from the group consisting of: SCF₃, SOCF₃, SO₂CF₃, SCF₂SO₂Ph, SOCF₂SO₂Ph, SO₂CF₂SO₂Ph, and SeCF₃.

### EXAMPLES

### Example 1: with alkyl amines

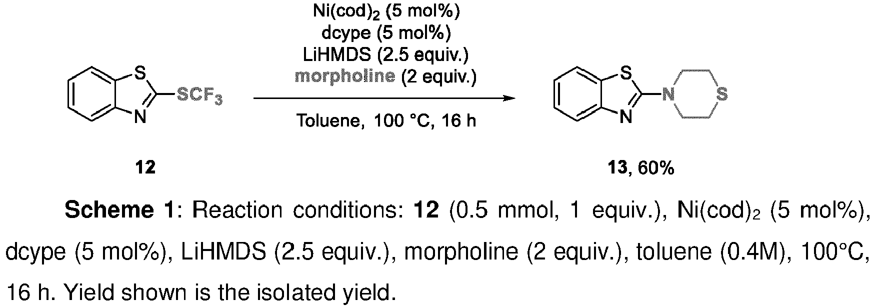

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), alkyl amine (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 2: with aryl amines

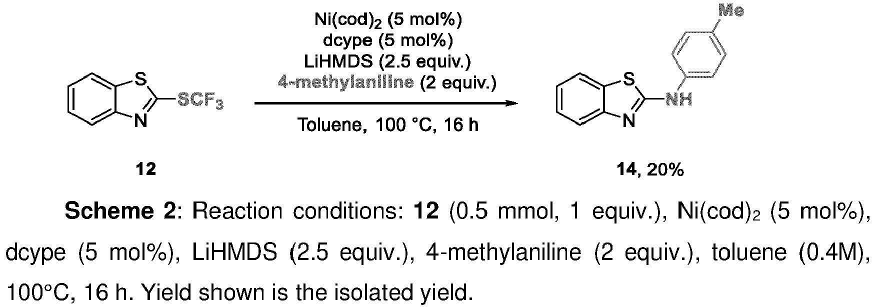

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), aryl amine (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 3: with alkyl thiol

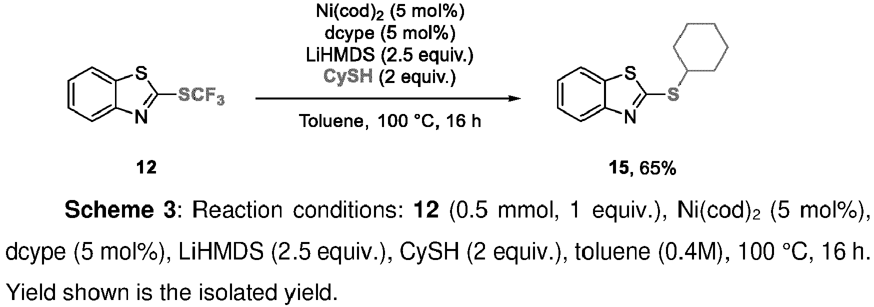

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), alkyl thiol (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 4: with thiophenol derivatives

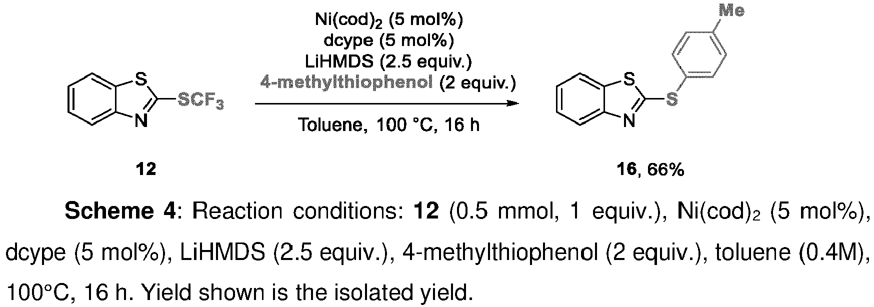

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), aryl thiol (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 5: with aryl selenol

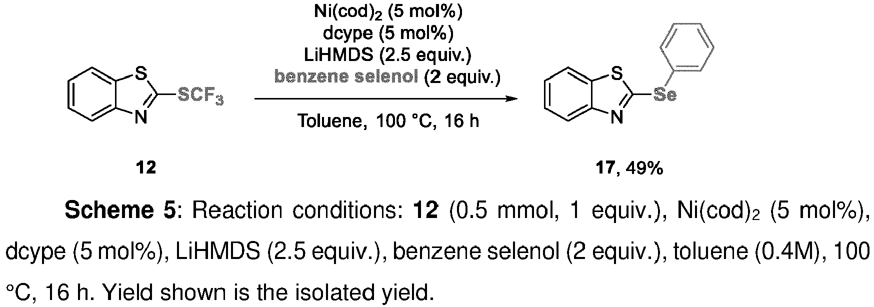

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), aryl selenol (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 6: with phosphine oxide:

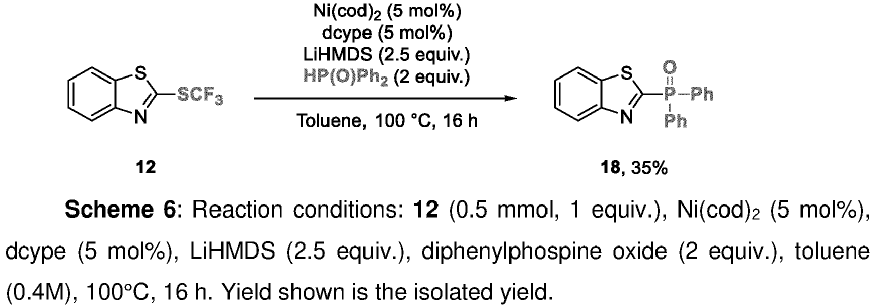

An oven-dried 10 mL tube equipped with a stirring bar was charged with Ni(cod)₂ (7 mg, 0.025 mmol, 5 mol%), dcype (11 mg, 0.025 mmol, 5 mol%), LiHMDS (209 mg, 1.25 mmol, 2.5 equiv.), 2-((trifluoromethyl)thio)benzo[*d*]thiazole **12** (118 mg, 0.5 mmol, 1 equiv.), phosphine oxide (1.25 mmol, 2 equiv.) and toluene (1.25 mL) under argon. The resulting solution was stirred at 100 °C for 16 h. The mixture was allowed to cooled down to 21 °C. EtOAc (10 mL) was added in the mixture and the resulting solution was washed with an aqueous solution of NaOH (1M, 10 mL). The aqueous phase was extracted with EtOAc (3 × 20 mL) and the combined organic phase were dried over MgSO4 and concentrated under vacuum. The residue was purified by silica gel flash column chromatography by dry loading the samples and eluting with a solvent system as noted below to afford the desired products.

### Example 7: with Grignard reagent

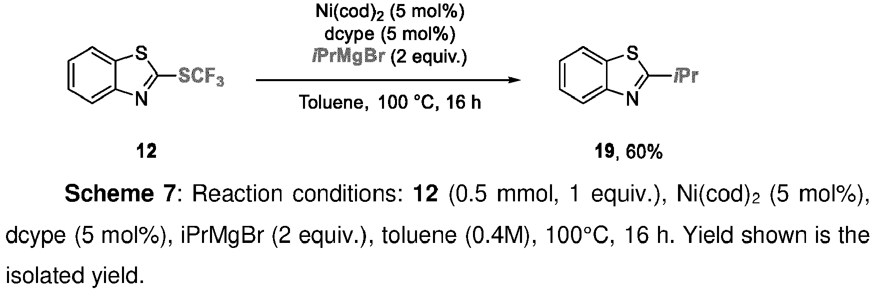

An oven-dried 10 mL tube equipped with a stirring bar was charged with Ni(cod)₂ (7 mg, 0.025 mmol, 5 mol%), dcype (11 mg, 0.025 mmol, 5 mol%), 2-((trifluoromethyl)thio)benzo[d]thiazole **12** (118 mg, 0.5 mmol, 1 equiv.), Grignard reagent (1.25 mmol, 2 equiv.) and toluene (1.25 mL) under argon. The resulting solution was stirred at 100 °C for 16 h. The mixture was allowed to cooled down to 21 °C. The residue was concentrated under vacuum and purified by silica gel flash column chromatography by dry loading the samples and eluting with a solvent system as noted below to afford the desired products.

## Claims

1. A process for the preparation of a compound having the formula (I):
A-R
by reacting a compound having the formula (II):
A-X
in the presence of a metallic complex comprising nickel, a ligand, a nucleophile carrying one group R, and a solvent,
R being a hydrocarbon group, optionally including at least one heteroatom and/or at least one atom other than C or H,
wherein:
- A is selected from the group consisting of:
. (C₆-C₁₀)aryl groups,
said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
Rₐ and R_{b} being independently from each other H or a (C₁-C₆)alkyl group;
R_{c} being a (C₁-C₆)alkyl group;
. heteroaryl groups comprising from 5 to 10 atoms and including at least one heteroatom selected from O, N, and S,
said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
. a vinyl compound having the following formula (III): 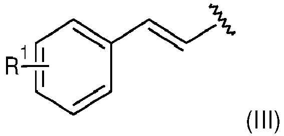 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
- X is selected from the group consisting of: YCF₃, YOCF₃, YO₂CF₃, YCF₂SO₂Ph, YOCF₂SO₂Ph, YO₂CF₂SO₂Ph, Y(O)ₙCF₂COOR', Y(O)ₙCF₂CONR'₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR', Y being S or Se,
n being 1 or 2, and R' being (C₁-C₆)alkyl.

2. The process of claim 1, wherein the nucleophile is selected from the group consisting of: alkyl amines, cycloalkyl amines, aryl amines, alkyl thiols, thiophenol compounds, aryl selenol compounds, phosphine oxides, and Grignard reagents.

3. The process of claim 1 or 2, wherein the nucleophile is a RH group, wherein R is selected from the group consisting of: (C₁-C₆)alkyl, heterocycloalkyl, -S-cyclo(C₃-C₁₀)alkyl, -P(=O)-R¹R², and -X¹-Ar¹, X¹ being S, Se or NH, and Ar¹ being an optionally substituted (C₆-C₁₀)aryl group, and R¹ and R² being, independently from each other, a (C₁-C₆)alkyl group or a (C₆-C₁₀)aryl group.

4. The process of any one of the preceding claims, wherein A is selected from the heteroaryl groups comprising from 5 to 10 atoms and including at least one heteroatom selected from O, N, and S, said heteroaryl groups being optionally substituted with at least one substituent selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined in claim 1.

5. The process of any one of the preceding claims, further comprising the use of a hydride compound, such as LiHMDS.

6. The process of any one of the preceding claims, wherein the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-tert-butylphosphino)ferrocene (DTBPF), 4,5-Bis(diphenylphosphino)-9,9-dimethylxanthene (XantPhos), and triphenylarsine (AsPh₃).

7. The process of any one of the preceding claims, wherein the solvent is toluene.

8. The process of any one of the preceding claims, wherein the reaction is carried out at a temperature from 90°C to 140°C.

9. The process of any one of the preceding claims, wherein the metallic complex is a Ni(0) complex such as bis(1,5-cyclooctadiene)nickel.

10. The process of any one of the preceding claims, for the preparation of a compound having the formula (I-1): 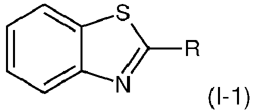
R being as defined above in any one of claims 1 to 3,
from a compound having the formula (II-1): 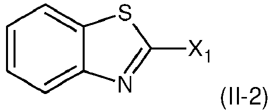 X₁ being selected from the group consisting of: SCF₃, SOCF₃, SO₂CF₃, SCF₂SO₂Ph, SOCF₂SO₂Ph, SO₂CF₂SO₂Ph, and SeCF₃.
